# EUROPEAN PATENT APPLICATION

(11) **EP 3 922 106 A1**
(43) Date of publication of application: **15.12.2021**
(21) Application number: 20752116.2
(22) Date of filing: 07.02.2020
(51) Int. Cl.: A23K 10/16, A23K 20/158, A23K 50/30, A23K 50/75

(54) **FUNCTIONAL FEED**

(30) Priority: 08.02.2019 JP 2019021520
(71) Applicant: Idemitsu Kosan Co., Ltd., Chiyoda-ku Tokyo, 100-8321 (JP)
(72) Inventor: HIKITA, Chie, Tsukuba-shi, Ibaraki 300-2646 (JP)
(74) Representative: Vossius & Partner Patentanwälte Rechtsanwälte mbB
(86) International application number: PCT/JP2020/004828
(87) International publication number: WO 2020/162599

(57) **Abstract**

A feed including cashew nut shell liquid and bacterial cells is provided for raising non-human monogastric animals such as chickens or pigs, wherein the cashew nut shell liquid content is not more than 500 ppm by mass with respect to the total amount of the feed. The feed can stimulate weight gain in non-human monogastric animals such as chickens or pigs and increase the efficiency in, for example, meat production.

## Description

### TECHNICAL FIELD

The present invention relates to a feed for non-human monogastric animals such as chickens or pigs, comprising cashew nut shell liquid and cells of a bacterium, and to a raising method using the same for non-human monogastric animals.

### BACKGROUND ART

Increased production and shipment of meat per unit farming area is a key factor in increasing the productivity of chickens for meat. Egg and meat productivity has already been largely enhanced to date by vaccination and improvement of farming systems as well as by genetic breeding, but further improvement of productivity is still demanded.

In some examples, the production efficiency is improved by improving nutritional care for chickens and, additionally, by feeding chickens with some functional substances, including antioxidants, viable cell preparations (for example, *Bacillus* spp., *Lactobacillus* spp., *Pseudomonas* spp., and *Flavobacterium* spp.), alkaloids (for example, piperine), enzymes (for example, phytase and protease), herbs (for example, common verbena, chameleon plant, garlic powder, allspice, and clove), amino acids, and monosaccharides. However, there still remains a need for other materials to further increase the productivity in the art of poultry farming.

Patent Document 1 (Japanese Unexamined Patent Publication No. H8-231410) describes a coccidiosis-relieving agent for poultry such as chickens for meat, wherein the coccidiosis-relieving agent comprises, for example, cashew nut shell liquid as an active ingredient.

Patent Document 2 (Japanese Unexamined Patent Publication No. 2001-151675) describes a feed for poultry such as chickens for meat to prevent and/or to treat coccidiosis, wherein the feed comprises, as active ingredients, cashew nut shell liquid and at least one ingredient selected from the group consisting of an organozinc compound, a betaine, and a *Bacillus* bacterium.

Patent Document 3 (Japanese Unexamined Patent Publication No. 2014-121331) has disclosed the effects of a feed containing cashew nut shell liquid at a concentration of 0.5% on reduction of *Clostridium perfringens* bacteria and on stimulation of weight gain, which are observed in parent broiler chickens infected with *Clostridium perfringens* and fed with the feed.

However, the effects of the agent and the feeds on unaffected healthy chickens are not indicated in those documents.

Patent Document 4 (WO 2016/121963) has disclosed that egg-laying chickens fed with a feed containing cashew nut shell liquid showed an increased egg production and a reduced death rate during molting period.

Patent Document 5 (WO 2017/138654) has disclosed that chickens for meat fed with a feed containing cashew nut shell liquid showed a reduced disposal rate and an increased productivity.

However, those documents have disclosed no specific embodiment with respect to feeding of cashew nut shell liquid in combination with viable cell preparations.

### PRIOR ART DOCUMENTS

### Patent Documents

Patent Document 1: Japanese Unexamined Patent Publication No. H8-231410
Patent Document 2: Japanese Unexamined Patent Publication No. 2001-151675
Patent Document 3: Japanese Unexamined Patent Publication No. 2014-121331
Patent Document 4: WO 2016/121963
Patent Document 5: WO 2017/138654

### SUMMARY OF THE INVENTION

### PROBLEMS TO BE SOLVED BY THE INVENTION

An object of the present invention is to provide a feed for raising non-human monogastric animals such as poultry including chickens and pigs healthily and for increasing the efficiency in, for example, meat production.

### MEANS FOR SOLVING THE PROBLEMS

The inventors conducted intensive studies to solve the above problem, and consequently found that feeding of a low concentration of cashew nut shell liquid, which is not higher than a certain concentration, in combination with cells of a bacterium, such as those of a *Bacillus* bacterium, for raising healthy non-human monogastric animals such as poultry including chickens and pigs, can stimulate weight gain in the non-human monogastric animals and eventually increase the feed efficiency as well as the efficiency in, for example, meat production. Thereby, the inventors have completed the present invention.

That is, the present invention is as follows.
(1) A feed for non-human monogastric animals, comprising cashew nut shell liquid and cells of a bacterium, wherein the cashew nut shell liquid content is not more than 500 ppm by mass with respect to the total amount of the feed.
(2) The feed according to (1), wherein the cashew nut shell liquid content is not more than 100 ppm by mass with respect to the total amount of the feed.
(3) The feed according to (1), wherein the cashew nut shell liquid content is not more than 50 ppm by mass with respect to the total amount of the feed.
(4) The feed according to any one of (1) to (3), wherein the bacterium is one or more species selected from the group consisting of spore-forming bacteria and lactic acid bacteria.
(5) The feed according to (4), wherein the bacterium is one or more species selected from the group consisting of the genera *Bacillus, Enterococcus, Lactobacillus,* and *Clostridium.*
(6) The feed according to (5), wherein the bacterium is one or more species selected from the group consisting of *Bacillus subtilis, Bacillus coagulans, Enterococcus faecium, Lactobacillus acidophilus,* and *Clostridium butyricum.*
(7) The feed according to any one of (1) to (6), wherein the content of the cells is 1×10⁴ to 1×10⁷ cells per g feed.
(8) The feed according to any one of (1) to (7), wherein the non-human monogastric animal is poultry or pig.
(9) The feed according to (8), wherein the poultry is chicken, quail, or turkey.
(10) The feed according to (9), wherein the poultry is chicken for meat.
(11) A method of producing a feed for non-human monogastric animals, comprising formulating cashew nut shell liquid and cells of a bacterium with feed materials, wherein the cashew nut shell liquid is formulated at a concentration of not more than 500 ppm by mass with respect to the total amount of the feed.
(12) A method of raising or fattening non-human monogastric animals, comprising feeding cashew nut shell liquid and cells of a bacterium to the non-human monogastric animals, wherein the cashew nut shell liquid is fed as a feed containing cashew nut shell liquid at a concentration of not more than 500 ppm by mass.
(13) A method of increasing the feed efficiency of a feed for non-human monogastric animals, comprising formulating cashew nut shell liquid and cells of a bacterium with feed materials, wherein the cashew nut shell liquid is formulated at a concentration of not more than 500 ppm by mass with respect to the total amount of the feed.
(14) A weight gain promoting agent to be formulated in a feed for non-human monogastric animals, comprising cashew nut shell liquid and cells of a bacterium, wherein the cashew nut shell liquid is formulated at a concentration of not more than 500 ppm by mass with respect to the total amount of the feed.
(15) A feed efficiency improving agent to be formulated in a feed for non-human monogastric animals, comprising cashew nut shell liquid and cells of a bacterium, wherein the cashew nut shell liquid is formulated at a concentration of not more than 500 ppm by mass with respect to the total amount of the feed.
(16) Use of cashew nut shell liquid and cells of a bacterium for stimulating weight gain in non-human monogastric animals, wherein the cashew nut shell liquid is contained and used in a feed for non-human monogastric animals at a concentration of not more than 500 ppm by mass with respect to the total amount of the feed.
(17) Use of cashew nut shell liquid and cells of a bacterium for improving the feed efficiency of a feed for non-human monogastric animals, wherein the cashew nut shell liquid is contained and used in the feed at a concentration of not more than 500 ppm by mass with respect to the total amount of the feed.

### ADVANTAGEOUS EFFECTS OF THE INVENTION

By using a feed according to the present invention, weight gain can be promoted in non-human monogastric animals such as chickens or pigs, which increases the efficiency in, for example, meat production. Additionally, cashew nut shell liquid at a predetermined concentration and cells of a bacterium contained in a feed for non-human monogastric animals can improve (increase) the feed efficiency.

### DETAILED DESCRIPTION OF THE INVENTION

The feed of the invention contains cashew nut shell liquid and cells of a bacterium (bacterial cells).

Cashew nut shell liquid is an oily liquid contained in shells of cashew (*Anacardium occidentale* L.) nuts. The components of cashew nut shell liquid include anacardic acid, cardanol, and cardol, and heat treatment converts anacardic acid to cardanol. Cashew nut shell liquid (unheated) extracted from cashew nut shells by pressing comprises 55 to 80% by mass of anacardic acid, 5 to 20% by mass of cardanol and 5 to 30% by mass of cardol, as described in J. Agric. Food Chem., 2001,49,2548-2551. Heat treatment of cashew nut shell liquid causes anacardic acid, the main component of cashew nut shell liquid, to be decarboxylated and converted to cardanol, and the resulting cashew nut shell liquid comprises 0 to 10% by mass of anacardic acid, 55 to 80% by mass of cardanol and 5 to 30% by mass of cardol. Moreover, storage of cashew nut shell liquid at room temperature (20°C) for about one year or longer can cause anacardic acid, the main component of cashew nut shell liquid, to be decarboxylated and converted to cardanol, and the resulting cashew nut shell liquid comprises 0 to 40% by mass of anacardic acid, 30 to 80% by mass of cardanol and 5 to 30% by mass of cardol.

The cashew nut shell liquid contained in the feed of the invention is not specifically limited, provided that the cashew nut shell liquid contains at least either anacardic acid or cardanol, and the cashew nut shell liquid may be either unheated cashew nut shell liquid or heated cashew nut shell liquid.

Cashew nut shell liquid can be obtained as a vegetable oil extracted from cashew nut shells by pressing. Moreover, cashew nut shell liquid can also be obtained from cashew nut shells by, for example, solvent extraction (for example, Japanese Unexamined Patent Publication No. H8-231410). In addition, cashew nut shell liquid may refer to pieces of shell and/or testa, containing cashew nut shell liquid, obtained by pulverizing/crushing cashew nut shells. The cashew nut testa is the thin skin between shell and kernel (seed) of cashew nut. Moreover, a commercial cashew nut shell liquid product may be used. Cashew nut shell liquid (unheated) obtained as described above can be heated at a temperature of not lower than 70°C, preferably not lower than 130°C, to obtain heated cashew nut shell liquid. The heated cashew nut shell liquid may refer to pieces of shell and/or testa, containing cashew nut shell liquid, obtained by pulverizing/crushing heated cashew nut shells.

The cashew nut shell liquid content in the feed is not more than 500 ppm by mass, preferably not more than 250 ppm by mass, more preferably not more than 100 ppm by mass, further preferably not more than 50 ppm by mass, and particularly preferably not more than 25 ppm by mass. The minimum content is not specifically limited, provided that the cashew nut shell liquid exerts a weight increasing effect, but the minimum content is, for example, not less than 1 ppm by mass, preferably not less than 5 ppm by mass, and more preferably not less than 10 ppm by mass. Additionally, the cashew nut shell liquid content in the feed preferably ranges from 1 to 500 ppm by mass, more preferably from 1 to 250 ppm by mass, still more preferably from 1 to 100 ppm by mass, further preferably from 1 to 50 ppm by mass, and particularly preferably from 1 to 25 ppm by mass.

In cases where oily cashew nut shells are directly used or are pulverized or crushed and then used, or cashew nut testae are used instead of cashew nut shell liquid, the amount of cashew nut shells or testae contained in the feed should be within the above range, which is converted based on the content of cashew nut shell liquid (CNSL) in shells or in testae (the content of CNSL in cashew nut shells is from 25 to 30% by mass, while the content of CNSL in cashew nut testae is from 0.5 to 3.0% by mass).

The bacterium whose cells are added in the feed of the invention is not limited to a specific bacterial species, provided that the bacterium will not adversely affect the health in non-human monogastric animals and can exert a stimulating effect on weight gain when the bacterial cells are fed together with cashew nut shell liquid. Examples of the bacterial species include species of spore-forming bacteria and of lactic acid bacteria.

Examples of the spore-forming bacteria include the following bacteria:
Bacteria in the genus *Bacillus,*
   *Bacillus subtilis, Bacillus coagulans, Bacillus cereus, Bacillus megaterium;*
Bacteria in the genus *Clostridium,*
   *Clostridium butyricum.*

Examples of the lactic acid bacteria include the following bacteria:
Bacteria in the genus *Lactobacillus, Lactobacillus acidophilus, Lactbacillus brevis, Lactbacillus gasseri, Lactobacillus buchneri, Lactobacillus bulgaricus, Lactobacillus delburvecki, Lactobacillus casei, Lactobacillus crispatus, Lactobacillus curvatus, Lactobacillus halivaticus, Lactobacillus pentosus, Lactobacillus plantarum, Lactobacilli paracasei, Lactobacillus rhamnosus, Lactobacillus salivarius, Lactobacillus sporogenes, Lactobacillus sakei, Lactobacillus fructivorans, Lactobacillus hilgardii, Lactobacillus reuteri, Lactobacillus fermentum;*
Bacteria in the genus *Bifidobacterium, Bifidobacterium bifidum, Bifidobacterium breve, Bifidobacterium infantis, Bifidobacterium lactis, Bifidobacterium longum, Bifidobacterium adolescentis, Bifidobacterium mongoliense, Bifidobacterium pseudolongum, Bifidobacterium thermophilum;*
Bacteria in the genus *Lactococcus, Lactococcus lactis;*
Bacteria in the genus *Pediococcus, Pediococcus acidilactici, Pediococcus pentosaceus;*
Bacteria in the genus *Leuconostoc, Leuconostoc mesenteroides, Leuconostoc oenos;*
Bacteria in the genus *Streptococcus, Streptococcus thermophilus;*
Bacteria in the genus *Enterococcus, Enterococcus faecium, Enterococcus faecalis.*

As the bacteria used for the feed of the invention, bacteria contained in, for example, commercially available viable cell preparations may be directly used, or bacterial cells obtained by culturing a bacterial strain may be used. The culture method for a bacterium is not specifically limited, and any known method suitable for each bacterium can be employed. For example, bacteria can be cultured in a proliferation medium, and bacterial cells can be recovered by means of, for example, centrifugation. In cases where spore-forming bacteria are used, spores from the spore-forming bacteria may be added.

The number of bacterial cells added to the feed of the invention is not specifically limited but should be an amount high enough to exert a stimulating effect on weight gain and is preferably from 1×10⁴ to 1×10⁷ cells, more preferably from 1×10⁵ to 1×10⁶ cells.

In order to allow the feed of the invention to include cashew nut shell liquid, cashew nut shell liquid, cashew nut shells, pulverized or crushed cashew nut shells, or cashew nut testae, the latter three of which also contain cashew nut shell liquid, may be directly mixed (blended) with feed materials to a cashew nut shell liquid concentration of not more than 500 ppm by mass, preferably not more than 250 ppm by mass, more preferably not more than 100 ppm by mass, still more preferably not more than 50 ppm by mass, with respect to the total amount of the feed, or cashew nut shell liquid may be adsorbed on carriers such as silica and formulated into a dosage form, and then mixed with feed materials to a cashew nut shell liquid concentration as described above. A technique for formulation of cashew nut shell liquid in combination with silica is described in, for example, WO 2009/151048. Also, a technique for formulation of cashew nut shell liquid using an oil absorbing agent, such as diatom earth, bentonite, montmorillonite, zeolite, perlite, acid clay, activated clay, or hydrated silica, is described in, for example, WO 2011/013592.

In order to allow the feed of the invention to contain bacterial cells, viable cell preparations or cultured bacteria can be directly mixed with feed materials, or a liquid or solid bacterial culture may be simply mixed with feed materials, to reach a predetermined cell number. Additionally, bacterial cells may be combined with necessary ingredients as appropriate, such as a stabilizing agent, nutritional ingredients, and carriers for the bacteria, and the resulting mixture is then mixed with feed materials.

Materials used for conventional feeds for non-human monogastric animals (for example, feeds for poultry (chicken farming), pigs (pig farming), and the like) can be used as feed ingredients (feed materials) other than cashew nut shell liquid and bacterial cells and may be appropriately selected according to the kinds of non-human monogastric animals.

Examples of the feed ingredients (feed materials) other than cashew nut shell liquid and bacterial cells include optional ingredients such as an ingredient effective in growth promotion, nutritional supplement ingredients, and an ingredient for enhancing storage stability. Examples of the optional ingredients include enzymes, such as amylase and lipase; vitamins, such as L-ascorbic acid, choline chloride, inositol, and folic acid; minerals, such as potassium chloride, ferric citrate, magnesium oxide, and phosphoric acid salts; amino acids, such as D,L-alanine, D,L-methionine, and L-lysine hydrochloride; organic acids, such as fumaric acid, butyric acid, lactic acid, and acetic acid, and salts thereof; antioxidants, such as ethoxyquin and dibutyl hydroxy toluene; fungicides, such as calcium propionate; thickeners, such as carboxymethyl cellulose (CMC), sodium caseinate, and sodium polyacrylate; emulsifiers, such as glycerin fatty acid esters and sorbitan fatty acid esters; pigments, such as asthaxanthin and canthaxanthin; and flavors, such as various esters, ethers, and ketones.

The feed of the invention may comprise a sugar (for example, lactose or trehalose), maize, milo, wheat bran, rice bran, defatted bran, dried bran, steam-rolled barley, steam-rolled corn, soybean cake, corn flour, rice flour, soybean flour, or the like.

Furthermore, water, vegetable oil such as soybean oil, rapeseed oil, or corn oil, liquid animal oil, and water-soluble high molecular compounds such as polyvinyl alcohol, polyvinylpyrrolidone, or polyacrylic acid may be used as a liquid carrier to facilitate the mixing of all the feed ingredients. Also, a water soluble polysaccharide such as alginic acid, sodium alginate, xanthan gum, sodium caseinate, gum arabic, guar gum, or tamarind seed polysaccharide is preferably blended to keep cashew nut shell liquid homogenously distributed in the feed.

Examples of the non-human monogastric animals to which the feed of the invention is fed includes poultry and pigs. Examples of the poultry include chickens, quails, and turkeys. The chickens may be egg-laying chickens or chickens for meat. The non-human monogastric animals are preferably healthy animals without any disease such as coccidiosis.

Examples of the chickens for meat include chickens of the White Cornish, White Plymouth Rock, Barred Plymouth Rock, Rhode Island Red, New Hampshire, UK Chunky, US Chunky, Cobb, Avian, Arbor Acres, Hinai-Jidori, Satsuma-Jidori, and Nagoya breeds.

Examples of the egg-laying chickens include chickens of the Julia, Julia-Lite, Babcock B400, Novogen White, Novogen Brown, and Sonia breeds. The egg-laying chickens may be not only the original or parent breeds of the above egg-laying chickens but also be commercial chicken breeds, crossbreeds, and improved breeds, which are developed from the above egg-laying chickens by genetic breeding programs. Examples of the egg-laying chickens include chickens of the White Leghorn, ISA Brown, Dekalb-Warren Sex-Sal-Link, Hubbard-Comet, Shaver Star Cross 579, Black Minorca, Barred Plymouth Rock, White Plymouth Rock, Rhode Island Red, New Hampshire, Araucana, Silky Fowl, Polish, Nagoya-Cochin, Hinai-Jidori, Boris Brown, Julia, Julia-Lite, Sonia, Maria, Laura, Novogen White, Novogen Brown, Elbe, Sakura, and Momiji breeds and may also include crossbreeds and improved breeds from the above breeds.

Examples of the quails include White Quail, Red Quail, King Quail, California Quail, Stripe-faced Wood Quail, Spot-winged Wood Quail, Long-tailed Wood Partridge, Mountain Quail, Northern Bobwhite, Grey Partridge, Montezuma Quail, Ocellated Quail, Scaled Quail, Common Quail, Yucatan Bobwhite, Crested Bobwhite, Gambel's Quail, Elegant Quail, Long-tailed Wood Partridge, Marbled Wood Quail, and Spotted Wood Quail.

Examples of the pigs include pigs of the Large White, Middle White, Landrace, Duroc, Berkshire, and Hampshire breeds and may also include crossbreeds and improved breeds from the above breeds.

The time of feeding suitable for the feed of the invention is not specifically limited, and the feed of the invention may be fed to chickens for meat or to pigs at any stage from birth to shipment or may be fed without interruption or for a fixed time period (for example, a total of 10 days or longer, preferably 20 days or longer). The feed of the invention can be fed to chickens for meat at any one or more stages selected from the group consisting of the early fattening stage, the late fattening stage, and the finishing stage. The feed of the invention may be fed to egg-laying chickens or to quails at any stage from hatch to egg laying or may be fed without interruption or for a fixed time period (for example, a total of 10 days or longer, preferably 20 days or longer).

The daily feeding amount suitable for the feed of the invention ranges, for example, from 10 to 1,000 g/day/head in poultry such as chickens and quails and from 50 to 7,000 g/day/head in pigs. The daily feeding amount of cashew nut shell liquid ranges, for example, from 0.000001 to 1.0 g/day/head in poultry such as chickens and quails and from 0.0005 to 5.0 g/day/head in pigs.

The raising method of the present invention for non-human monogastric animals is characterized by feeding non-human monogastric animals with the feed of the invention; however, cashew nut shell liquid and bacterial cells, the active ingredients of the feed of the invention, may be individually fed to the animals. That is, in one embodiment of the raising method of the present invention for non-human monogastric animals, a feed containing cashew nut shell liquid at a concentration of not more than 500 ppm by mass and a feed containing bacterial cells at a predetermined concentration are individually fed to the animals. In this case, a feed containing cashew nut shell liquid at a concentration of not more than 500 ppm by mass and a feed containing bacterial cells at a predetermined concentration may be fed separately at different times.

### EXAMPLES

The present invention will be specifically described below by way of example, but the invention is not limited to the aspects of the following examples.

### Production Example

Cashew nut shell liquid (CNSL) was purchased from Thao Nguyen Co., Ltd. The composition of the CNSL was determined by the method below. That is, a HPLC system (Waters 600, Nihon Waters K.K.), a detector (Waters 490E, Nihon Waters K.K.), a printer (Chromatopac C-R6A, Shimadzu Corp.) and a column (SUPELCOSIL LC18, Supelco) were used. A solvent of acetonitrile/water/acetic acid (80:20:1, vol/vol/vol) was used at a flow rate of 2 mL/min. Detection was performed by absorbance at 280 nm. It was found that the cashew nut shell liquid contained 65.7% by mass of anacardic acid, 5.1% by mass of cardanol and 23.5% by mass of cardol.

### Example 1

UK Chunky broiler chicks without any disease were used, and the chicks, seven in each group, were fed with mixed diets during the course of test, during which the body weight was measured at the time of introduction (1 day old), at 3 days old, at 10 days old, and at 17 days old. The standard laboratory diet for early-age broiler chickens SDB No.1 (manufactured by Feed One Co., Ltd.) was used as a basal diet, and cultured cells of the *Bacillus subtilis* strain DB9011 (FERM BP-3418; WO2008/023580) were used as the bacterial cells. The bacterial cells and CNSL were mixed with the basal diet to a cell density of 3×10⁵ cells/g and to each concentration (25 ppm by mass, 50 ppm by mass, or 100 ppm by mass), respectively, to produce test diets. The no-addition group, in which both the bacterial cells and CNSL were not added to the basal diet, and the bacterial cell group, in which only the bacterial cells were added to the basal diet, were established as controls.

The result is shown in Table 1. The daily consumption amount was, on average, 8.5 g/head/day in the period from 1 day old to 3 days old, 30.5 g/head/day in the period from 3 days old to 10 days old, and 63.0 g/head/day in the period from 10 days old to 17 days old, and the average consumption amount in the period of 17 days was 40 g/head/day. That is, in the group in which CNSL was added to the basal diet to a concentration of 25 ppm, the daily consumption amount of CNSL was 213 µg/head/day in the period from 1 day old to 3 days old, 763 µg/head/day in the period from 3 days old to 10 days old, and 1,574 µg/head/day in the period from 10 days old to 17 days old.

**Table 1 Results of body weight (g) measurement in each group**

| | 1day old | 3days old | 10days old | 17days old | Weight gain rate at 17 days old |
|---|---|---|---|---|---|
| No addition | 45.4 | 62.4 | 217.8 | 571.5 | |
| B. subtilis only | 44.7 | 59.8 | 237.0 | 576.9 | 0.95% |
| B. subtilis+CNSL 25 ppm | 45.7 | 65.1 | 219.1 | 584.9 | 2.34% |
| B. subtilis+CNSL 50 ppm | 45.0 | 63.4 | 243.6 | 582.7 | 1.96% |
| B.subtilis+CNSL 100 ppm | 45.9 | 64.4 | 241.6 | 581.5 | 1.76% |

| | | | | | |
|---|---|---|---|---|---|
| * Cells of the *Bacillus subtilis* strain DB9011 was mixed at a cell density of 3.0×10⁵ cells/g feed. | | | | | |

As seen in Table 1, a significant weight gain was observed from Day 10 in each of the groups fed with a diet containing the bacterial cells and CNSL, and the weight gain rate was more significant in the groups in which the basal diet was mixed with CNSL at a concentration of 25 ppm by mass and of 50 ppm by mass.

### Example 2

UK Chunky broiler chicks without any disease were used, and the chicks, seven in each group, were fed with mixed diets during the course of test. The standard laboratory diet for early-age broiler chickens SDB No.1 (manufactured by Feed One Co., Ltd.) was used as a basal diet, and a no-addition control diet, a diet containing CNSL, and a diet containing CNSL and cells of different bacterial species were produced. Commercial feed-additive-grade viable cell preparations of different bacterial species were used as the bacterial cells. In each diet, the concentration of the added CNSL was 25 ppm, and the amount of the added cells of different bacterial species was 5.0×10⁵ cfu/g. The no-addition group, in which both the bacterial cells and CNSL were not added to the basal diet, and the CNSL group, in which only CNSL was added to the basal diet, were established as controls.

Feeding of each diet was started from the time of introduction of the animals into each group and was continued until the age of 28 days. Body weight was measured at the time of introduction and at 28 days old to calculate weight gain and to calculate feed conversion rate from the feed consumption amount in each group. The result is shown in Table 2.

**Table 2 Evaluation for the use of various microbial cells**

| Added substances | Average body weight (g) | | Average weight gain (g) | Feed conversion rate |
|---|---|---|---|---|
| | 0 day old | 28 days old | | |
| Control (no addition) | 47.8 | 1489.6 | 1441.8 | 1.41 |
| CNSE | 47.8 | 1497.7 | 1449.9 | 1.38 |
| CNSE+Bacillus subtilis | 47.8 | 1506.5 | 1458.7 | 1.49 |
| CNSE+Lactobacillus acidophilus | 47.8 | 1504.3 | 1456.5 | 1.45 |
| CNSE+Enterococcus feacium | 47.8 | 1515.3 | 1467.5 | 1.44 |
| CNSE+Clostridium butyricum | 47.8 | 1501.7 | 1453.9 | 1.43 |
| CNSE+Bacillus coagulans | 47.9 | 1525.8 | 1477.9 | 1.43 |

As shown in Table 2, an increased average weight gain and an improved feed conversion rate were observed in each of the groups fed with a diet containing CNSL. Furthermore, the weight gain was found to be higher in each group fed with a diet containing CNSL and bacterial cells of different species than in the group fed with a diet containing only CNSL as an additive.

### INDUSTRIAL APPLICABILITY

The feed of the present invention is useful in the field of, for example, chicken and livestock farming.

## Claims

1. A feed for non-human monogastric animals, comprising cashew nut shell liquid and cells of a bacterium, wherein the cashew nut shell liquid content is not more than 500 ppm by mass with respect to the total amount of the feed.

2. The feed according to claim 1, wherein the cashew nut shell liquid content is not more than 100 ppm by mass with respect to the total amount of the feed.

3. The feed according to claim 1, wherein the cashew nut shell liquid content is not more than 50 ppm by mass with respect to the total amount of the feed.

4. The feed according to any one of claims 1 to 3, wherein the bacterium is one or more species selected from the group consisting of spore-forming bacteria and lactic acid bacteria.

5. The feed according to claim 4, wherein the bacterium is one or more species selected from the group consisting of the genera *Bacillus, Enterococcus, Lactobacillus,* and *Clostridium.*

6. The feed according to claim 5, wherein the bacterium is one or more species selected from the group consisting of *Bacillus subtilis, Bacillus coagulans, Enterococcus faecium, Lactobacillus acidophilus,* and *Clostridium butyricum.*

7. The feed according to any one of claims 1 to 6, wherein the content of the cells is 1×10⁴ to 1×10⁷ cells per g feed.

8. The feed according to any one of claims 1 to 7, wherein the non-human monogastric animal is poultry or pig.

9. The feed according to claim 8, wherein the poultry is chicken, quail, or turkey.

10. The feed according to claim 9, wherein the poultry is chicken for meat.

11. A method of producing a feed for non-human monogastric animals, comprising formulating cashew nut shell liquid and cells of a bacterium with feed materials, wherein the cashew nut shell liquid is formulated at a concentration of not more than 500 ppm by mass with respect to the total amount of the feed.

12. A method of raising non-human monogastric animals, comprising feeding cashew nut shell liquid and cells of a bacterium to the non-human monogastric animals, wherein the cashew nut shell liquid is fed as a feed containing cashew nut shell liquid at a concentration of not more than 500 ppm by mass.

13. A method of increasing the feed efficiency of a feed for non-human monogastric animals, comprising formulating cashew nut shell liquid and cells of a bacterium with feed materials, wherein the cashew nut shell liquid is formulated at a concentration of not more than 500 ppm by mass with respect to the total amount of the feed.
